# EUROPEAN PATENT APPLICATION

(11) **EP 3 020 404 A1**
(43) Date of publication of application: **18.05.2016**
(21) Application number: 14382450.6
(22) Date of filing: 14.11.2014
(51) Int. Cl.: A61K 31/522, A61P 43/00, A61P 21/00

(54) **Caffeine for the treatment of myotonic dystrophy type 1 and type 2**

(71) Applicant: Universitat de Valéncia, 46010 Valencia (ES); Institut Químic de Sarriá CETS Fundació Privada, 08017 Barcelona (ES); Institut Univ. de Ciència i Tecnologia, S.A., 08100 Mollet del Vallès Barcelona (ES)
(72) Inventor: Artero Allepuz, Ruben, 46010 València (ES); Castells Boliart, Josep, 08100 MOLLET DEL VALLES (ES); Borrell Bilbao, José Ignacio, 08017 Barcelona (ES); Llamusi Troísi, Beatriz, 46010 VALÈNCIA (ES); Bargiela Schönbrunn, Ariadna, 46010 VALÈNCIA (ES); Konieczny, Piotr, 46010 VALÈNCIA (ES); Pascual Gilabert, Marta, 08100 MOLLET DEL VALLÈS (ES); Teixidó Closa, Jordi, 08017 BARCELONA (ES); Estrada Tejedor, Roger, 08017 BARCELONA (ES); López González, Alejandro, 08017 BARCELONA (ES)
(74) Representative: Oficina Ponti, SLP

(57) **Abstract**

The present invention relates to caffeine for use in the treatment of myotonic dystrophy type 1 and type 2. The present invention also relates to compositions comprising caffeine for use in the treatment of myotonic dystrophy type 1 and type 2.

## Description

### Field of invention

The present invention relates to the field of rare diseases, in particular the invention relates to myotonic dystrophy. More particularly, the present invention relates to caffeine and compositions comprising thereof for use in the treatment of myotonic dystrophy type 1 and type 2.

### Background of the invention

Myotonic Dystrophy (DM) is the most common form of muscular dystrophy in adults and includes two clinically similar diseases although originating from distinct genetic mutations. DM1 (Steinert's disease) corresponds to the majority of the cases of DM and stems from an expansion of the CTG trinucleotide repeat (typically more than 50 units) in the 3'-untranslated region (UTR) of the *DMPK gene* whereas DM2 originates from big expansions of the CCTG tetranucleotide in the first intron of the *CNBP* gene. Both are rare diseases, with a global combined prevalence estimated at 12.5 per 100000, although it has been suggested that it could be three times higher (Suominen, T. et al. Population frequency of myotonic dystrophy: higher than expected frequency of myotonic dystrophy type 2 (DM2) mutation in Finland. Eur J Hum Genet. 19(7):776-82, 2011). DM1 is a multisystemic disease that affects primarily to the skeletal muscle (miotonia, muscle weakness and degeneration), the heart and the central nervous system (Gomes-Pereira, M. et al. Myotonic dystrophy mouse models: towards rational therapy development. Trends Mol Med. 17(9):506-17 (2011)). A large proportion of DM1 patients suffer heart conduction defects that can progress to full atrioventricular block or ventricular arryhtmias and sudden death in 30% of the patients. Cognitive alterations such as low processing speed, hypersomnia, and anhedonia prevent patients from an appropriate social interaction. DM1 can occur in patients of any age whereas DM2 typically presents in adults. Both DM1 and DM2 patients can also be considered as a person who carries the expanded CTG or CCTG mutation, respectively.

Cells carrying few CTG repeats show a functional equilibrium between two antagonistic splicing regulators: muscleblind-like 1 (MBNL1) and CUGBP/Elav-like family member 1 (CELF1). The balance between MBNL1 and CELF1 controls the establishment of adult splicing profiles for a subset of developmentally regulated transcripts. The CTG repeat expansion expressed in a DM1 cell forms an imperfect double-stranded stem loop structure that has two main pathogenic consequences: MBNL1 sequestration by RNA foci and protein kinase C (PKC)-mediated and AKT-mediated CELF1 hyperphosphorylation, stabilisation and subcellular redistribution. As a result of MBNL1 depletion and CELF1 upregulation, the balance between these two splicing regulators is disturbed and the alternative splicing of a series of developmentally regulated transcripts reverts to a foetal pattern. The abnormal expression of splicing isoforms in adult skeletal muscle, heart and brain is likely to contribute to the DM1 disease symptoms. Whereas MBNL1 seems critical in skeletal and cardiac phenotypes, sequestration of the MBNL2 paralog has been suggested to originate brain symptoms and MBNL3 may inhibit muscle differentiation. In addition to defective alternative splicing regulation, protein translation deficits, Repeat-Associated non-ATG translation (RAN translation) and bidirectional transcription of the *DMPK* gene, altered expression of microRNAs, gene transcription deficiencies, and RNA interference, among others, have been shown to contribute to molecular alterations typical of DM in different cell and animal models of the disease.

Current therapeutic strategies are based on candidate drugs that bind to CUG repeats, thereby releasing MBNL proteins to regulate splicing of its pre-mRNA targets. Then, the consensus strategy to reverse splicing abnormalities observed in DM1 is based in a reduction in nuclear foci and the concomitant reduction in nuclear sequestration of MBNL proteins. Warf and colleagues (Warf, M., Nakamori, M., Matthys, C., Thornton, C. and Berglund, J. (2009) Pentamidine reverses the splicing defects associated with myotonic dystrophy. Proceedings of the National Academy of Sciences of the United States of America, 106, 18551-18556. C. Thornton, C. and Berglund, J. (2009) Pentamidine reverses the splicing defects associated with myotonic dystrophy. Proceedings of the National Academy of Sciences of the United States of America, 106, 18551-18556. Coonrod, L., et al. (2013) Reducing levels of toxic RNA with small molecules. ACS Chem Biol. 8(11):2528-37) demonstrated that pentamidine facilitates a partial rescue of DM1 pathology.

Since then, some other therapies have been disclosed, such as antisense oligonucleotide compounds (see, EP 2 560 001 A2, US 2011/0269665 A1), pentamidine analogues (see, A., Haley, M., Thornton, C. et al. (2013) Reducing levels of toxic RNA with small molecules. ACS chemical biology, 8, 2528-2537; Parkesh, R., Childs-Disney, J., Nakamori, M., Kumar, A., Wang, E., Wang, T., Hoskins, J., Tran, T., Housman, D., Thornton, C. et al. (2012) Design of a Bioactive Small Molecule That Targets the Myotonic Dystrophy Type 1 RNA via an RNA Motif-Ligand Database and Chemical Similarity Searching. Journal of the American Chemical Society, 134, 4731-4742), peptides (see, EP 2 554 180 A1, Gareiss, P., Sobczak, K., McNaughton, B., Palde, P., Thornton, C. and Miller, B. (2008) Dynamic combinatorial selection of molecules capable of inhibiting the (CUG) repeat RNA-MBNL1 interaction in vitro: discovery of lead compounds targeting myotonic dystrophy (DM1). Journal of the American Chemical Society, 130, 16254-16261; Garcia-Lopez, A., Llamusi, B., Orzaez, M., Perez-Paya, E. and Artero, R. (2011) In vivo discovery of a peptide that prevents CUG-RNA hairpin formation and reverses RNA toxicity in myotonic dystrophy models. Proceedings of the National Academy of Sciences of the United States of America, 108, 11866-11871), or chemical drug candidates (see, US 8.754.084 B2, EP 2 742 974 A1, US 8.741.572 B1, US 2014/0051709 A1, Jahromi, A.H., Nguyen, L., Fu, Y., Miller, K.A., Baranger, A.M. and Zimmerman, S.C. (2013) A novel CUG(exp). MBNL1 inhibitor with therapeutic potential for myotonic dystrophy type 1. ACS chemical biology, 8, 1037-1043; Wong, C.H., Nguyen, L., Peh, J., Luu, L.M., Sanchez, J.S., Richardson, S.L., Tuccinardi, T.,Tsoi, H., Chan, W.Y., Chan, H.Y. et al. (2014) Targeting Toxic RNAs that Cause Myotonic Dystrophy Type 1 (DM1) with a Bisamidinium Inhibitor. Journal of the American Chemical Society, 136, 6355-6361; Childs-Disney, J., Stepniak-Konieczna, E., Tran, T., Yildirim, I., Park, H., Chen, C., Hoskins, J., Southall, N., Marugan, J., Patnaik, S. et al. (2013) Induction and reversal of myotonic dystrophy type 1 pre-mRNA splicing defects by small molecules. Nature communications, 4, 2044; Hoskins, J.W., Ofori, L.O., Chen, C.Z., Kumar, A., Sobczak, K., Nakamori, M., Southall, N., Patnaik, S., Marugan, J.J., Zheng, W. et al. (2014) Lomofungin and dilomofungin: inhibitors of MBNL1-CUG RNA binding with distinct cellular effects. Nucleic acids research, 42, 6591-6602).

However, although DM1 was first described in 1909, there is presently no cure or specific treatment for myotonic dystrophy. All the treatments applied are palliative and contribute to control the development of symptoms, and the clinical focus is on managing the complications of the disease, particularly those relating to the cardiopulmonary system as this account for 70% deaths due to DM1, but in no case for treating the disease in a definitive manner.

WO03/020977 relates to a method for identifying a substance capable of modulating the number of nucleotide repeats in a cell displaying expanded nucleotide repeat instability. This expansion of unstable nucleotide sequences is a primary genetic defect associated with DM1. This document states that caffeine is not able to bind to CUG repeats and increases the number of foci and consequently it is not a suitable candidate for drugs for treating myotonic dystrophy type 1 (DM1). See also Gomes-Pereira et al. 2004, Nuc. Acids Research, 2004, 32 (9), 2865-2872.

Nevertheless, the present inventors have focused the research on the grounds that overexpressing Muscleblind-like proteins, particularly MBNL1, has the potential of being therapeutic for this disease (see, Kanadia RN et al. Proc. Natl. Acad. Sci. USA 2006 Aug 1; 103 (31): 11748-53 and Chamberlain, C.M. et al. Human Molecular Genetics, 2012, 21(21), 4645-4654). The inventors have surprisingly found that, unlike any other xanthine derivative compounds, caffeine is able to increase the transcription of the human *MBNL1* promoter under the control of a muscle-specific *Drosophila muscleblind* (*mbl*) enhancer in transgenic flies, increasing thereby the expression of a enhanced Green Fluorescence Protein (eGFP) reporter. Importantly, caffeine was also able to increase the amount of free MBNL1 in human DM1 myoblasts.

An increase in free MBNL1 protein has been shown to reduce the severity of symptoms in animal models of DM1, while in contrast, a decrease of free MBNL1 protein is observed with longer expansions, which are correlated with more severe disease. The pathogenic role of the *MBNL1* gene is further substantiated by the fact that *MBNL1* gene variants modify DM1 severity (Vincent Huin et al. MBNL1 gene variants as modifiers of disease severity in myotonic dystrophy type 1. J Neurol (2013) 260:998-1003). Similarly, MBNL proteins have been shown to get sequestered in DM2, thus, strategies aimed at increasing steady state levels of these proteins have the potential to become treatments for DM2.

Therefore, according to the surprising finding by the present inventors, caffeine is considered a serious candidate for treating myotonic dystrophy type 1 (DM1) and myotonic dystrophy type 2 (DM2).

### Summary of the invention

A first aspect of the present invention relates to caffeine for use in the treatment of myotonic dystrophy type 1 and type 2

A second aspect of the present invention relates to compositions comprising caffeine for use in the treatment of myotonic dystrophy type 1 and type 2.

### Brief description of the drawings

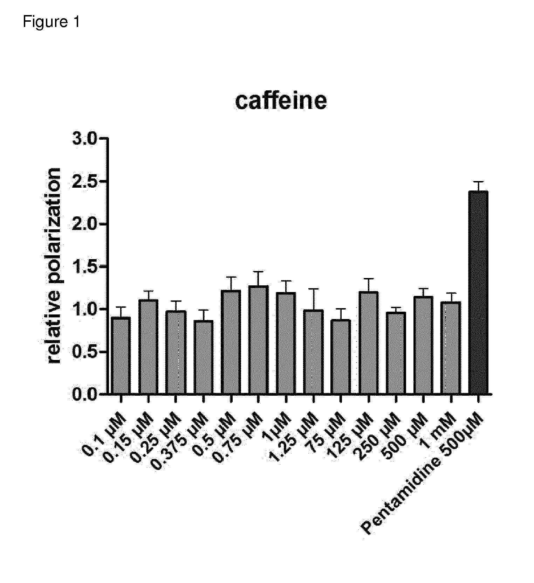

Figure 1 shows that caffeine does not bind CUG repeat RNA in fluorescence polarization assays. Bar graph shows polarization measurements relative to control alone from three independent experiments with four technical replicates each. Pentamidine was used as positive control. Error bars are s.e.m

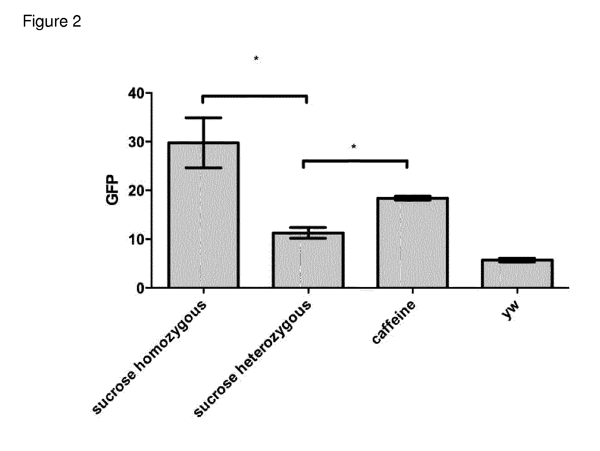

Figure 2 represents the quantification of eGFP expression from *yw* (negative control), homozygous (control of response to gene dosage) and heterozygous flies carrying the *ME:hsaP1-eGFP* construct, normalized to total amount of protein, taking caffeine or sucrose (dilutant). * indicates P<0.05. Data was generated from three biological replicates in arbitrary units of fluorescence. Error bars are s.e.m

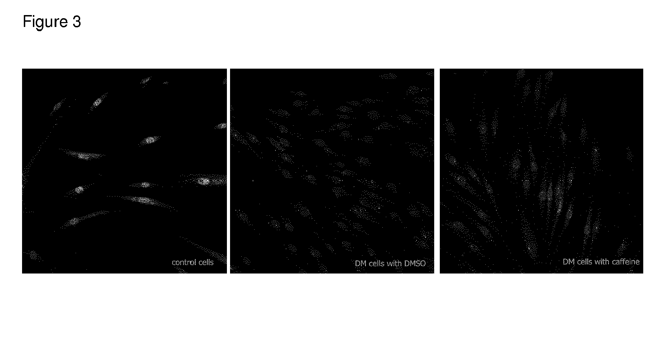

Figure 3: Confocal microscope images of normal and DM1 myoblasts showing expression of the MBNL1 protein (green channel, cell nuclei are counterstained with DAPI in the blue channel). In normal myoblasts MBNL1 is detected preferentially in the cell nucleus, although it is also found in the cytoplasm. In DM1 myoblasts incubated with DMSO, as control, MBNL1 is characteristically sequestered in ribonuclear foci. Addition of 125 µM caffeine to the cell medium for two days increased the amount of MBNL1 that could be detected both in the cytoplasm and in the ribonuclear foci.

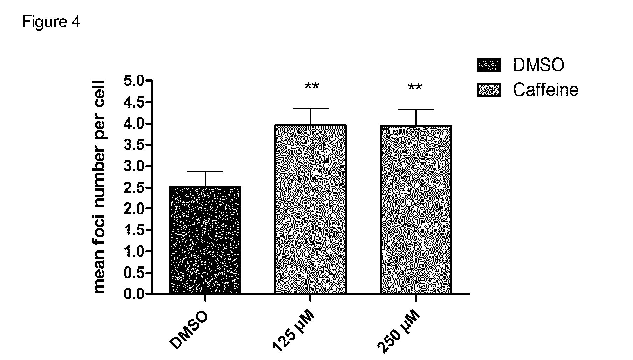

Figure 4: Mean number of ribonuclear foci per cell in DM1 fibroblasts exposed to the indicated concentrations of caffeine in the culture media. Results show that caffeine increased the number of foci per cell. ** indicates p-value < 0.001. Data comes from three independent experiments with three technical replicates each. DMSO bar shows reference value for DM1 fibroblasts exposed to dilutant only. Error bars are s.e.m.

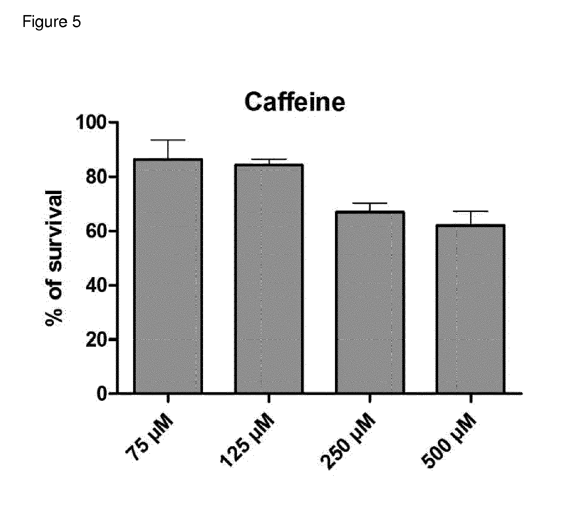

Figure 5: Percentage of survival of normal fibroblasts exposed to increasing concentrations of caffeine in the cell media. Even at the highest concentration the percentage of living cells was greater than 50%, thus caffeine is well tolerated by human fibroblasts. Data comes from three independent experiments. Error bars are s.e.m.

### Detailed description of the invention

The present invention relates to a compound which is caffeine 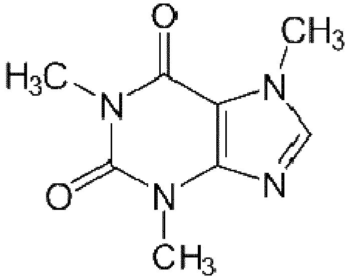 for use in the treatment of myotonic dystrophy type 1 or type 2.

In a particular embodiment, said compound is in the form of a tautomer, solvate, hydrate or a pharmaceutically acceptable salt thereof.

As used in the foregoing and hereinafter, the following definitions apply unless otherwise noted.

The present invention is also intended to include all isotopes of atoms occurring on the present compounds. Isotopes include those atoms having the same atomic number but different mass numbers. By way of general example and without limitation, isotopes of hydrogen include tritium and deuterium. Isotopes of carbon include C-13 and C-14.

The term "tautomer" or "tautomeric form" refers to structural isomer of different energies which are interconvertible via a low energy barrier. For example, proton tautomers (also known as prototropic tautomers) include interconversion via migration of a proton, such as keto-enol and imine-enamine isomerizations. Valence tautomers include interconversions by reorganization of some of the bonding electrons.

The present disclosure also includes the prodrugs leading to caffeine.

The term "prodrug" as used throughout this text means the pharmacologically acceptable derivatives such as esters, amides, and phosphates, such that the resulting *in vivo* biotransformation product of the derivative is the active drug as defined herein (caffeine). The reference by Goodman and Gilman (The Pharmacological Basis of Therapeutics, 8th ed, McGraw-Hill, Int. Ed. 1992, "Biotransformation of Drugs", p 13-15) describing prodrugs generally is hereby incorporated. Prodrugs preferably have excellent aqueous solubility, increased bioavailability and are readily metabolized into the active inhibitors *in vivo.* Prodrugs of caffeine may be prepared by modifying functional groups present in the compound in such a way that the modifications are cleaved, either by routine manipulation or *in vivo,* to the parent compound.

For therapeutic use, salts of caffeine are those wherein the counter-ion is pharmaceutically acceptable. "Pharmaceutically acceptable" as used herein means that the extract, fraction thereof, or compound thereof or composition is suitable for administration to a subject to achieve the treatments described herein, without unduly deleterious side effects in light of the severity of the disease and necessity of the treatment. However, salts of acids and bases which are non-pharmaceutically acceptable may also find use, for example, in the preparation or purification of a pharmaceutically acceptable compound. All salts, whether pharmaceutically acceptable or not are included within the ambit of the present invention.

The pharmaceutically acceptable acid and base addition salts as mentioned hereinabove are meant to comprise the therapeutically active non-toxic acid and base addition salt forms which caffeine is able to form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic (i.e. hydroxybutanedioic acid), tartaric, citric, methanesulfonic, ethanesulfonic, benzenesulfonic, p-toluenesulfonic, cyclamic, salicylic, p-aminosalicylic, pamoic and the like acids.

Conversely said salt forms can be converted by treatment with an appropriate base into the free base form.

The present invention also relates to a composition comprising caffeine for use in the treatment of myotonic dystrophy type 1 and type 2.

In a particular embodiment of said composition, the compound caffeine is in the form of a tautomer, solvate, hydrate or a pharmaceutically acceptable salt thereof.

In another embodiment of said composition, the composition further comprises at least a xanthine or a derivative thereof, wherein said derivative is selected from the group consisting of theophylline, theobromine, aminophylline, 1,7-dimethylxanthine, 3-isobutyl-1-methylxanthine, 3-methylxanthine, 3-ethyl-1-propylxanthine, 3-allyl-1-ethyl-8-hydroxyxanthine, 3,8-dimethyl-2-thioxanthine, 1-ethyl-3-isobutylxanthine, 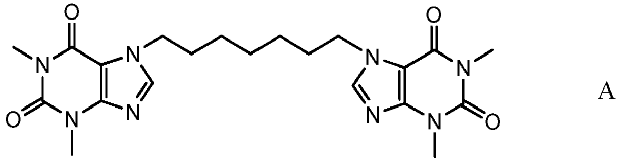 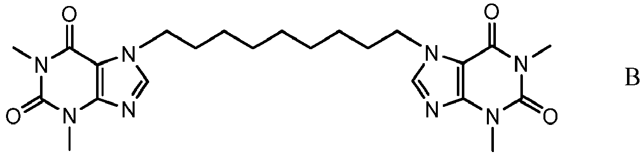 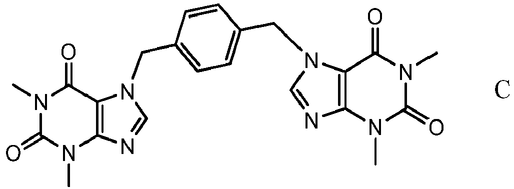 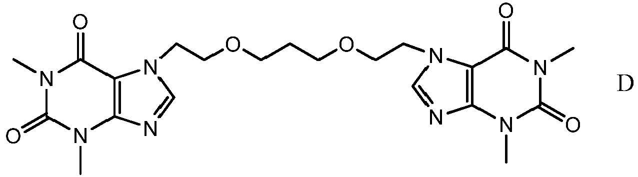 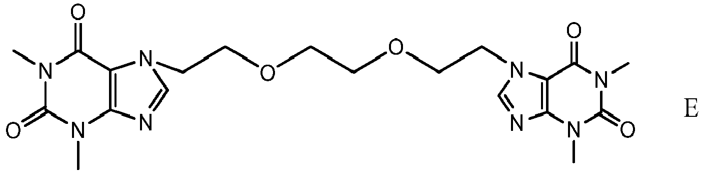 and 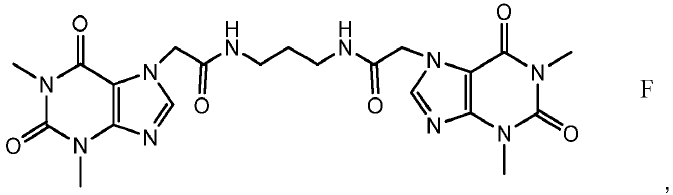 preferably said derivative is theophylline or theobromine.

The present invention also relates to a method of treatment of myotonic dystrophy type 1 and type 2 in a subject comprising administering to said subject a therapeutically effective amount of the compound which is caffeine or a tautomer, solvate, hydrate or a pharmaceutically acceptable salt thereof. Preferably the subject is a human subject. In addition, the present invention also relates to a method of treatment of myotonic dystrophy type 1 or type 2 in a subject comprising administering to said subject a therapeutically effective amount of a composition comprising caffeine or a tautomer, solvate, hydrate or a pharmaceutically acceptable salt thereof. The phrase "therapeutically effective amount" means that amount of such a substance that produces some desired local or systemic effect at a reasonable benefit/risk ratio applicable to any treatment. The therapeutically effective amount of such substance will vary depending upon the subject and disease condition being treated, the weight and age of the subject, the severity of the disease condition, the manner of administration and the like, which can readily be determined by one of ordinary skill in the art. For example, certain compositions of the present disclosure may be administered in a sufficient amount to produce a reasonable benefit/risk ratio applicable to such treatment.

As used herein, a patient or subject who will suffer from myotonic dystrophy type 1 or 2 is defined as a person who carried any of the gene mutations disclosed in the background section, even though the disease has not developed.

In another preferred embodiment, the composition for use according to the present invention is formulated as a pharmaceutical composition, food, food ingredient or supplement, nutraceutical composition, additive for a natural product or is present in the extract of a natural product. Preferably, said composition is the form of a solid or liquid. More preferably, said composition is present in a dairy product, a beverage or cereals.

A composition of a "food" item or "food ingredient or supplement" as described above may in principle take any form suited for consumption by man or animal. In one embodiment the composition is in the form of a dry powder that can be suspended, dispersed, emulsified or dissolved in an aqueous liquid such as water, coffee, tea, milk, yogurt, stock or fruit juice and alcoholic drinks. To this end, the powder may be provided in unit-dosage form. In an alternative preferred embodiment the composition in the form of a dry powder is tabletted. To that end, a composition for a food supplement according to the invention may very suitably be provided with fillers, such as microcrystalline cellulose (MCC) and mannitol, binders such as hydroxypropylcellulose (HPC), and lubricants such as stearic acid or other excipients. A composition of a food item or food supplement as described above may also be provided in the form of a liquid preparation wherein the solids are suspended, dispersed or emulsified in an aqueous liquid. Such a composition may be admixed directly through a food item or may e.g. be extruded and processed to grains or other shapes. In an alternative embodiment a food item or food supplement may take the shape of a solid, semi-solid or liquid food item, such as cereals, a bread, a bar, a cookie, a sandwich or a beverage, or as a spread, sauce, butter, margarine, dairy product, and the like. Preferably, the composition is included in a dairy product, such as for instance a butter or margarine, custard, yogurt, cheese, spread, drink, or pudding or other dessert.

Nutraceuticals can be defined as natural products that are used to supplement the diet by increasing the total dietary intake of important nutrients. This definition includes nutritional supplements such as vitamins, minerals, herbal extracts, antioxidants, amino acids, and protein supplements. Nutraceutical products fit into the newly created product category of "Dietary Supplements" as established by the F.D.A. in the Dietary Supplement Act of 1994. This act specifically defined dietary supplements to include: vitamins, minerals, herbs or other botanicals, antioxidants, amino acids, or other dietary substances used to supplement the diet by increasing the total daily intake. A "nutraceutical composition" is defined herein as a food composition fortified with ingredients capable of producing health benefits. Such a composition in the context of the present invention may also be indicated as foods for special dietary use; medical foods; and dietary supplements. For example, the food item or supplement may help to prevent or reduce symptoms associated with an inflammatory condition such as allergies (e.g. hay fever) and the like. As with the pharmaceutical composition, the amount of active ingredient in the food or food additive will depend on several factors. The food product will generally comprise a concentration that is sufficient to provide a consumer with an effective amount of active ingredient upon consumption of a regular (e.g. daily) portion of the food product. It will be recognized by those skilled in the art that the optimal quantity and spacing of individual dosages for achieving the therapeutic effects of the pharmaceutical composition, food item or food supplement described herein may easily be determined by the skilled person.

As mentioned above, the compounds or compositions disclosed herein can also be present in natural products. For example, it is well known that caffeine is found in varying quantities in the seeds, leaves, and fruit of some plants. It is for example extracted from the seed of the coffee plant and the leaves of the tea bush, as well as from various foods and drinks containing products derived from the kola nut. Other sources include yerba mate, guarana berries, guayusa, and the yaupon holly.

In another preferred embodiment, the compound or composition disclosed herein is administered by oral, rectal, nasal, topical, vaginal, parenteral, transdermal, intraperitoneal, intrapulmonary and intranasal route.

Dose ranges of the pharmaceutical compositions can be adjusted as necessary for the treatment of individual patients and according to the specific condition treated. Any of a number of suitable pharmaceutical formulations may be utilized as a vehicle for the administration of the compositions of the present invention and maybe a variety of administration routes are available. The particular mode selected will depend of course, upon the particular formulation selected, the severity of the disease, disorder, or condition being treated and the dosage required for therapeutic efficacy. The methods of this invention, generally speaking, may be practiced using any mode of administration that is medically acceptable, meaning any mode that produces effective levels of the active compounds without causing clinically unacceptable adverse effects. Such modes of administration include oral, rectal, topical, nasal, transdermal or parenteral routes and the like. Accordingly, the formulations of the invention include those suitable for oral, rectal, topical, buccal, sublingual, parenteral (e.g., subcutaneous, intramuscular, intradermal, inhalational or intravenous) and transdermal administration, although the most suitable route in any given case will depend on the nature and severity of the condition being treated and on the nature of the particular active product used.

Formulations suitable for oral administration may be presented in discrete units, such as capsules, cachets, lozenges, drops, or tablets, each containing a predetermined amount of the active compound; as a powder or granules; as a solution or a suspension in an aqueous or non-aqueous liquid; or as an oil-in-water or water-in-oil emulsion. Such formulations may be prepared by any suitable method of pharmacy which includes the step of bringing into association the active compound and a suitable carrier (which may contain one or more accessory ingredients as noted above).

In general, the formulations of the invention are prepared by uniformly and intimately admixing the active compound with a liquid or finely divided solid carrier, or both, and then, if necessary, shaping the resulting mixture. For example, a tablet may be prepared by compressing or molding a powder or granules containing the active compound, optionally with one or more accessory ingredients. Compressed tablets may be prepared by compressing, in a suitable machine, the compound in a free-flowing form, such as a powder or granules optionally mixed with a binder, lubricant, inert diluent, and/or surface active/dispersing agent(s). Molded tablets may be made by molding, in a suitable machine, the powdered compound moistened with an inert liquid binder.

Formulations of the present invention suitable for parenteral administration conveniently comprise sterile aqueous preparations of the active compound, which preparations are preferably isotonic with the blood of the intended recipient. These preparations may be administered by means of subcutaneous, intravenous, intramuscular, inhalational or intradermal injection. Such preparations may conveniently be prepared by admixing the compound with water or a glycine buffer and rendering the resulting solution sterile and isotonic with the blood.

Formulations of the inventive mixtures are particularly suitable for topical application to the skin and preferably take the form of an ointment, cream, lotion, paste, gel, spray, aerosol, or oil. Carriers which may be used include Vaseline, lanoline, polyethylene glycols, alcohols, transdermal enhancers, and combinations of two or more thereof.

Formulations suitable for transdermal administration may also be presented as medicated bandages or discrete patches adapted to remain in intimate contact with the epidermis of the recipient for a prolonged period of time. Formulations suitable for transdermal administration may also be delivered by iontophoresis (passage of a small electric current to "inject" electrically charged ions into the skin; also called electromotive drug administration (EMDA)) through the skin.

The present invention is now further illustrated by reference to the following examples.

### EXAMPLES

### Caffeine cannot bind CUG expansions in fluorescence polarization spectroscopy experiments

This was assessed in fluorescence polarization spectroscopy experiments in which fluorescent CUG RNA probe (carboxyfluorescein (6-FAM)-labeled) was incubated in increasing concentrations of caffeine. Whereas 6-FAM-CUG RNA molecules do not fluorescence in any particular polarization axis, binding to a molecule slows down the rotational movement of the molecule and increases polarization values.

**Results for caffeine:** caffeine did not increase polarization values of 6-FAM-CUG RNA even at concentrations twice those employed with the positive control pentamidine, which suggest that caffeine was unable to bind to the CUG repeats and therefore can not prevent sequestration of MBNL proteins in ribonuclear foci.

### See fig. 1.

### Materials and Methods

### Polarization fluorescence assays

Carboxyfluorescein-labeled CUG RNA (23 CUG repeats; 6-FAM-CUG₂₃) at 6 nM was incubated with caffeine at different concentrations in binding buffer (50 mM Tris-HCl pH 7.0, 250 mM NaCl, 50 µM ZnCl₂, 10% glycerol, 1 mM DTT) on ice for 20 min in the dark. Polarization was measured in a EnVision® Multilabel Reader using as excitation filter FP480 and as emission filter FP535.

### Caffeine increases free Muscleblind protein

### • Evidences from Drosophila:

We have previously characterized the transcriptional regulation of the *muscleblind* gene in *Drosophila* (Bargiela et al. Two enhancers control transcription of Drosophila muscleblind in the embryonic somatic musculature and in the central nervous system. 2014 Mar 25;9(3):e93125) and have identified a defined genomic regulatory region able to drive the expression of *muscleblind* in all somatic muscles ("ME" enhancer region) and a minimal promoter from the human *MBNL1* ortholog (*hsaP1*). In particular, ME placed upstream of the endogenous *MBNL1* promoter and of the enhanced Green Fluorescence Protein (eGFP) reporter expressed eGFP throughout the *Drosophila* somatic musculature in transgenic flies *(ME:hsaP1-eGFP* flies).

Flies heterozygous for the *ME:hsaP1-eGFP* construct were used in a chemical screen searching for compounds capable of modifying the expression of the reporter and thus, potentially, transcription of the *muscleblind* locus. Caffeine was found to significantly increase the expression of the eGFP reporter. Therefore, it is inferred that caffeine has the ability to increase transcription of the endogenous *muscleblind* gene.

### See fig. 2.

### Materials and Methods

*ME:hsaP1-eGFP* flies were crossed to *yw* to obtain heterozygous offspring. 2-day-old heterozygous flies were used for *in vivo* testing of compounds, serving *yw* and homozygous flies as controls of background and increased reporter expression, respectively. 25 mg/ml of caffeine (Sigma Aldrich) was dissolved in standard fly food (final concentration of the sucrose dilutant in food, 10%). Five flies per tube were used in each test and the experiment was carried out for 8 h at 25°C. Flies with the same genotype but taking sucrose 10% in standard food served as controls. Three biological replicates per group were performed. To measure eGFP, three flies were homogenized and eGFP was measured using a Tecan plate reader. Total amount of protein was quantified using the BCA protein assay kit (Pierce) and was used to normalize eGFP measurements.

### • Caffeine increases MBNL1 in human DM1 myoblasts

To investigate whether caffeine had an effect on MBNL1 expression levels in human cells we immunodetected MBNL1 in normal and control DM1 myoblasts, and in DM1 myoblasts grown in 125 and 250 µM caffeine. At both concentrations MBNL1 expression increased in the cytoplasm and in the cell nucleus compared to DM1 myoblasts exposed to DMSO alone (solvent of caffeine). The observed increase of MBNL1 expression could originate from a number of ways: release from ribonuclear foci, increase in transcription or enhanced protein stability. However, the number of MBNL1-containing foci in caffeine-treated cells was qualitatively higher than in DMSO-treated cells, which is not consistent with a model of release from CUG expansions. Thus, caffeine increases expression of MBNL1. Because the levels of MBNL1 in DM1 myoblasts are limiting, an increase in their levels, even if ribonuclear foci increase, is potentially therapeutic.
See fig. 3.
See fig. 4.

### Materials and Methods

### Cell culture conditions

Cell model of the disease (provided by the D. Furling's laboratory, Institute of Myologie, Paris) consisted of normal and DM1 (1300 CTG repeats) immortalized (hTERT) skin fibroblasts expressing conditional MyoD. Fibroblast cells were grown in Dulbecco's Modified Eagle Medium (DMEM) with 4.5 g/L of glucose, 1 % of penicillin and streptomycin (P/S) and 10% foetal bovine serum (FBS) (Sigma). Fibroblasts were transdifferentiated in myoblasts by inducing expression of MyoD. Cells were plated in muscle differentiation medium (MDM) made of DMEM 4.5 g/L glucose with 1% P/S, 2% horse serum, 1% apo-transferrin (10 mg/ml), 0,1 % insulin (10 mg/ml) and 0,02 % doxycyclin (10 mg/ml) for 48 h. For compound testing fibroblasts were aliquoted in 24-well plate with 3,5 x 10⁴ cells per well and were differentiated as before. Caffeine (Sigma-Aldrich) was added to a final concentration of 125 µM and 250 µM in MDM medium and cells were incubated for 48 h.

### Immunodetection of MBNL1

Cells were fixed in 4% paraformaldehyde (PFA) for 10 min at room temperature followed by several washes in PBS 1x. Cells were then permeabilized with 0.3 % Triton in PBS (PBT), blocked (PBT 1% donkey serum) for 30 min at room temperature, and incubated with primary antibody (mouse anti-MBNL1 1:500; Sigma) at 4°C overnight. After several washes with PBT cells were incubated for 45 min with biotin-conjugated secondary antibody (Sigma) at a 1:200 dilution. Cells were then incubated with ABC solution (ABC kit, VECTASTAIN) for 30 min at room temperature, followed by PBT washes and incubation with streptavidin-FITC (1:1000) for 20 min. Samples were finally mounted in Vectashield (Vector) with 2 µg/ml DAPI.

### Foci detection

*In situ* hybridization with CUG repeat RNA. Cells were fixed in 4% paraformaldehyde (PFA) for 10 min at room temperature followed by several washes in PBS 1x. Fixed cells were incubated in pre-hybridization buffer (SSC 2x, 30% deionized formamide) for 10 min at room temperature, hybridized with Cy3-(CAG)₇-Cy3 labelled probe diluted 1:100 in hybridization buffer (40% formamide, 2x SSC, 0.2% BSA, 10% dextran sulfate, 2 mM vanadyl complex, 10% tRNA (10 mg/ml), 10% herring sperm) for 2 h at 37°C, washed twice in pre-hybridization buffer for 15 min at 45°C, washed in PBS 1x for 15 min at room temperature and mounted in Vectashield (Vector) with 2 µg/ml DAPI. Images were taken using a Leica DM2500 fluorescence microscope and foci were manually counted from at least 50 cells per compound.
See fig. 4

### Toxicity studies

### Reference is made to figure 5.

### Materials and Methods

Fibroblast cells were grown in DMEM with 4.5g/L of glucose, 1% of penicillin and streptomycin (P/S) and 10% foetal bovine serum (FBS) (Sigma). Cells were aliquoted in 96-well plate with 1.0 x 10⁴ cells per well and incubated for 24 h. Caffeine (Sigma-Aldrich) was added to a final concentration of 75 µM, 125 µM, 250 µM and 500 µM in DMEM and cells were incubated for 24 h. To measure cell viability, MTS tetrazolium salt was added to each well and was incubated for 4 h at 37°C in a humidified chamber with 5% CO₂. The conversion of MTS into soluble formazan (accomplished by dehydrogenase enzymes from metabolically active cells) was measured by absorbance at 490 nm (CellTiter 96® Aquₑₒᵤₛ Non-Radioactive Cell Profileration Assay, Promega). Data were transformed to percentage of survival relative to cells not exposed to caffeine, which established 100% viability.

Here we report the surprising finding that caffeine promotes higher levels of MBNL1 in human DM1 myoblasts (Fig. 3) and boosts expression of a reporter of the *Drosophila melanogaster* homologue gene in transgenic animals (Fig. 2). Furthermore, caffeine does not bing to CUG repeat RNA according to polarization spectroscopy assays (Fig. 1) and increases the mean number of foci per myoblast in a cell model of the disease (Fig. 4). Finally, caffeine is particularly well tolerated by human fibroblasts in cell culture conditions (Fig. 5). Taken together these data are consistent with caffeine being able to promote expression of MBNL1 in human cells, instead of releasing MBNL1 from CUG foci, as previously reported for other small molecules. Because there is ample evidence that MBNL1 activity is limiting in DM1 and DM2 diseases due to sequestration by CUG and CCUG repeat expansions, that strategies aimed at increasing the amount of free MBNL1 are therapeutic in cell and animal experimentation models, and that MBNL1 overexpression is well-tolerated in skeletal muscle and is compatible with life, we propose that caffeine can be used for the treatment of DM1 and DM2 in patients.

## Claims

1. A compound which is caffeine 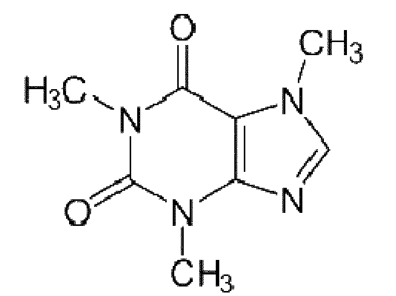 for use in the treatment of myotonic dystrophy type 1 and type 2.

2. Compound for use, according to claim 1, which is in the form of a tautomer, solvate, hydrate or a pharmaceutically acceptable salt thereof.

3. A composition comprising a compound which is caffeine 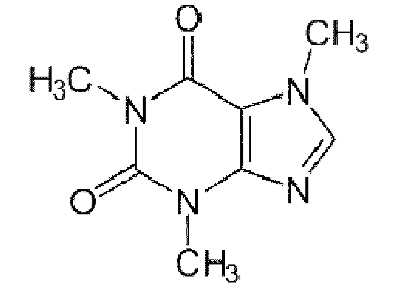 for use in the treatment of myotonic dystrophy type 1 and type 2.

4. Composition for use, according to claim 4, wherein said compound which is caffeine is in the form of a tautomer, solvate, hydrate or a pharmaceutically acceptable salt thereof.

5. Composition for use according to claim 3 or 4, further comprising at least a xanthine or a derivative thereof, wherein said derivative is selected from the group consisting of theophylline, theobromine, aminophylline, 1,7-dimethylxanthine, 3-isobutyl-1-methylxanthine, 3-methylxanthine, 3-ethyl-1-propylxanthine, 3-allyl-1-ethyl-8-hydroxyxanthine, 3,8-dimethyl-2-thioxanthine, 1-ethyl-3-isobutylxanthine, 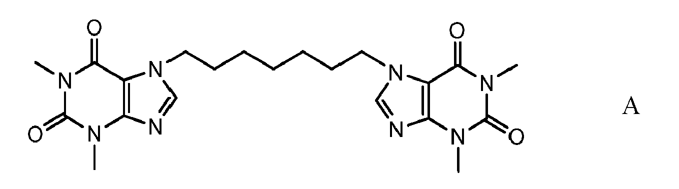 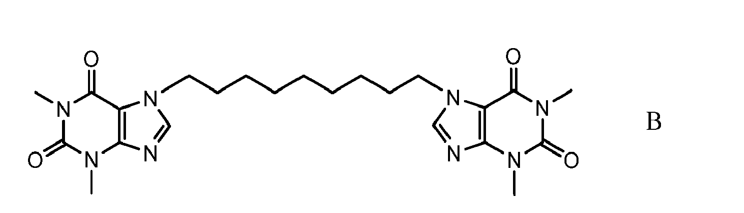 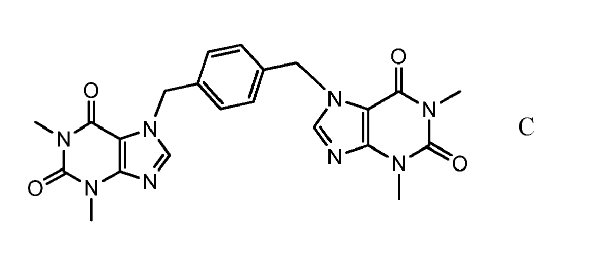 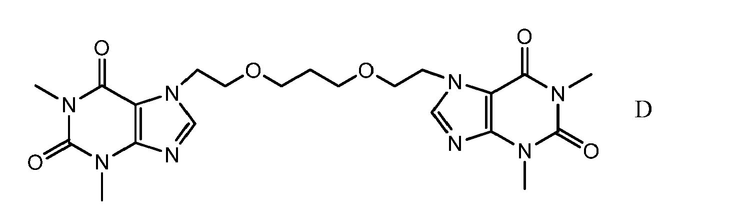 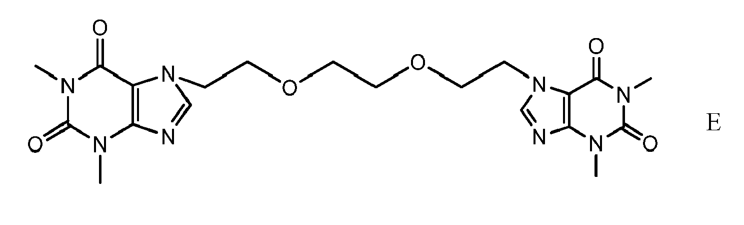 and

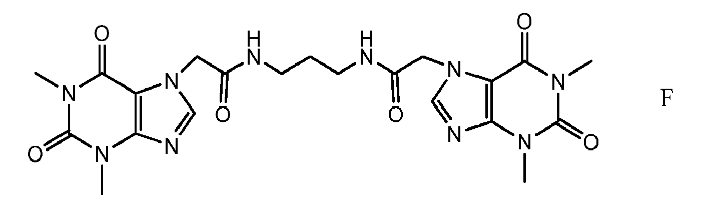

6. Composition for use, according to any of claims 3 to 5, which is formulated as a pharmaceutical composition, food, food ingredient or supplement, nutraceutical composition, additive for a natural product or is present in the extract of a natural product.

7. Composition for use according to claim 6, wherein said composition is the form of a solid or liquid.

8. Composition for use, according to any of claims 6 or 7, wherein said composition is present in a diary product, a beverage or cereals.

9. Compound for use, according to any of claims 1 or 2, or composition, according to any of claims 3 to 8, which is administered by oral, rectal, nasal, topical, vaginal, parenteral, transdermal, intraperitoneal, intrapulmonary and intranasal route.
